# EUROPEAN PATENT APPLICATION

(11) **EP 2 832 397 A1**
(43) Date of publication of application: **04.02.2015**
(21) Application number: 13768435.3
(22) Date of filing: 25.03.2013
(51) Int. Cl.: A61M 25/14, A61M 25/092, A61M 25/095

(54) **MEDICAL INSTRUMENT AND METHOD FOR MANUFACTURING MEDICAL INSTRUMENT**

(30) Priority: 26.03.2012 JP 2012069043
(71) Applicant: Sumitomo Bakelite Co., Ltd., Shinagawa-ku Tokyo 140-0002 (JP)
(72) Inventor: KANEMASA, Kenichi, Akita-shi Akita 011-8510 (JP); TANAKA, Hayao, Akita-shi Akita 011-8510 (JP); ABE, Yoshihiro, Akita-shi Akita 011-8510 (JP)
(74) Representative: Müller-Boré & Partner Patentanwälte PartG mbB
(86) International application number: PCT/JP2013/058562
(87) International publication number: WO 2013/146673

(57) **Abstract**

Provided is a medical instrument which can reliably protect a sub-lumen and a method of manufacturing the medical instrument. In such a medical instrument, a catheter (100) includes a resin tubular main body (sheath) (10), in which a main lumen (20) and a sub-lumen (80) which is disposed on an outer peripheral side of the main lumen (20) and extends along a longitudinal direction of the main lumen (20), are formed; a reinforcement layer (30) which is disposed between the main lumen (20) and the sub-lumen (80) and surrounds the main lumen (20); and a wire (91) which is disposed further on an outer peripheral surface side of the tubular main body (10) than the sub-lumen (80) and is disposed so as to surround the outer periphery of the main lumen (20). The wire (91) is embedded in the tubular main body (10) and supports the outer peripheral surface of the tubular main body (10).

## Description

### Technical Field

The present invention relates to a medical instrument and a method of manufacturing the medical instrument.

Priority is claimed on Japanese Patent Application No. 2012-069043, filed March 26, 2012, the content of which is incorporated herein by reference.

### Background Art

In recent years, a catheter through which it is possible to operate the approach direction to the lumen of a living body by bending a distal end portion has been provided. For example, PTL 1 discloses a catheter which is provided with a wire lumen (sub-lumen) in a periphery of a main lumen inside of a tube, and is formed of a pushing and pulling wire inside of the sub-lumen. In the catheter, a tip end of the catheter is bent by operating the pushing and pulling wire.

### Citation List

### Patent Literature

[PTL 1] Published Japanese Translation No. 2007-507305 of the PCT International Publication

### Summary of Invention

### Technical Problem

In recent years, in order to improve an insertion property of a catheter into the lumen of a living body such as a blood vessel, reducing the wall thickness of a tube formed with a main lumen and a sub-lumen has been reviewed.

In the catheter disclosed in PTL 1, when the wall thickness of the above-described tube is made thin, the wall thickness of the portion at an outer peripheral side of the catheter becomes thinner than the sub-lumen. For this reason, there is a concern that the sub-lumen enters a state of not being sufficiently protected by resin constituting the tube.

### Solution to Problem

According to the present invention, there is provided a medical instrument including: a resin tubular main body, in which a main lumen and a sub-lumen which is disposed on an outer peripheral side of the main lumen and extends along a longitudinal direction of the main lumen, are formed; a reinforcement layer which is disposed between the main lumen and the sub-lumen and surrounds the main lumen; and a first wire which is disposed further on an outer peripheral surface side of the tubular main body than the sub-lumen and is disposed so as to surround the sub-lumen and the main lumen. The first wire is embedded in the tubular main body and supports the outer peripheral surface of the tubular main body.

According to the present invention, the first wire is disposed further on the outer peripheral surface side of the tubular main body than the sub-lumen. Moreover, the first wire is embedded in the tubular main body and supports the outer peripheral surface of the tubular main body. The outer peripheral surface is elevated by the first wire supporting the outer peripheral surface of the tubular main body, and therefore, it is possible to protect the thickness of a portion positioned further on the outer peripheral side of the tubular main body than the sub-lumen, on the tubular main body. Accordingly, it is possible to protect the sub-lumen.

Furthermore, according to the present invention, it is possible to provide a method of manufacturing the above-described medical instrument.

That is, according to the present invention, there is provided a method of manufacturing a medical instrument including: a step of preparing a molded body having a main body portion which is a tubular resin main body and in which a main lumen formation region and a sub-lumen formation region which is disposed on an outer peripheral side of the main lumen formation region and extends along a longitudinal direction of the main lumen formation region, are formed, and a reinforcement layer which is disposed between the main lumen formation region and the sub-lumen formation region and surrounds the main lumen formation region; a step of disposing a first wire on an outer peripheral side of the main body portion of the molded body so as to surround the molded body; and a step of embedding the first wire in the tubular main body such that the wire supports the outer peripheral surface of the tubular main body by pressurizing the molded body and the first wire from a radial direction of the main lumen formation region of the main body portion and by heating the molded body and the first wire.

### Advantageous Effects of Invention

According to the present invention, there is provided a medical instrument which can reliably protect the sub-lumen and a method of manufacturing the medical instrument.

### Brief Description of Drawings

Fig. 1 is an enlarged view of a cross section of a catheter according to an embodiment of the present invention.
Fig. 2 is a cross-sectional view in a II-II direction of Fig. 1.
Fig. 3(a) and 3(b) are partial enlarged views of the cross-sectional view in the II-II direction of Fig. 1.
Fig. 4 is an enlarged view showing a modification example.
Fig. 5 is a side surface view of the catheter when viewed from a direction orthogonal to a longitudinal direction of the catheter.

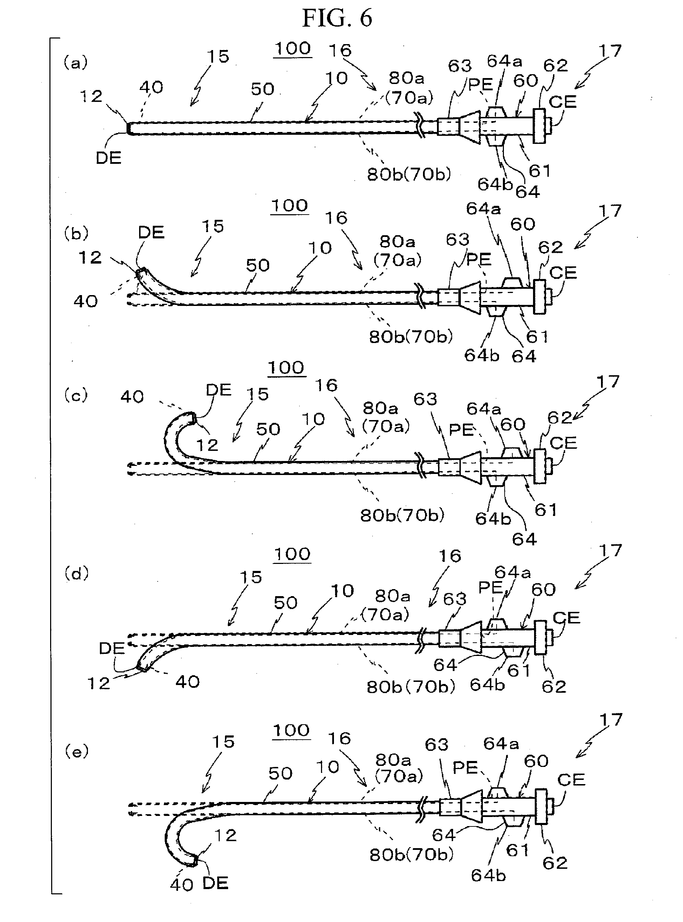

Fig. 6 is a side surface view showing an entire catheter and an example of a tip end portion being bent. (a) is a side surface view showing the entire catheter before being bent, (b) is a side surface view showing a state in which a slider is operated to bend the tip end upward, (c) is a side surface view showing a state in which the slider is operated to bend the tip end upward at a greater curvature than (b), (d) is a side surface view showing a state in which the slider is operated to bend the tip end downward, and (e) is a side surface view showing a state in which the slider is operated to bend the tip end downward at a greater curvature than (d).
Fig. 7 is a cross-sectional view showing a process of manufacturing the catheter.
Fig. 8 is a view according to a second embodiment of the present invention. (a) is a plan view showing a wire with tape and (b) is a cross-sectional view in a b-b direction of (a).
Fig. 9 is a side surface view according to the second embodiment of the present invention when viewed from a direction orthogonal to the longitudinal direction of a catheter.

### Description of Embodiments

Hereinafter, the embodiments of the present invention will be described with reference to the drawings. In all drawings, components having the same configuration are given of the same reference numerals and the detailed description will be appropriately omitted to avoid repetition.

### (First Embodiment)

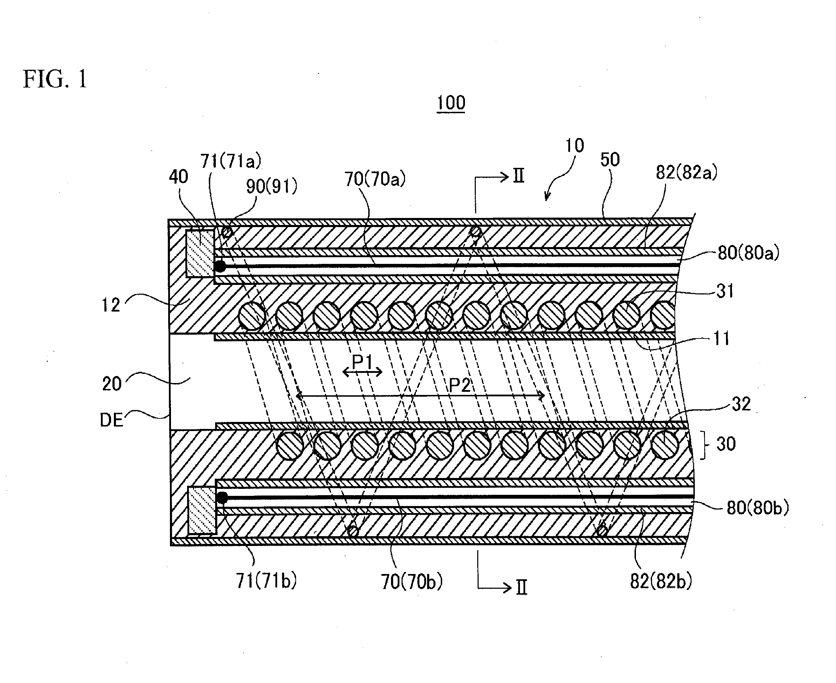

An embodiment of the present invention will be described with reference to Figs. 1 to 7.

### [Configuration Example]

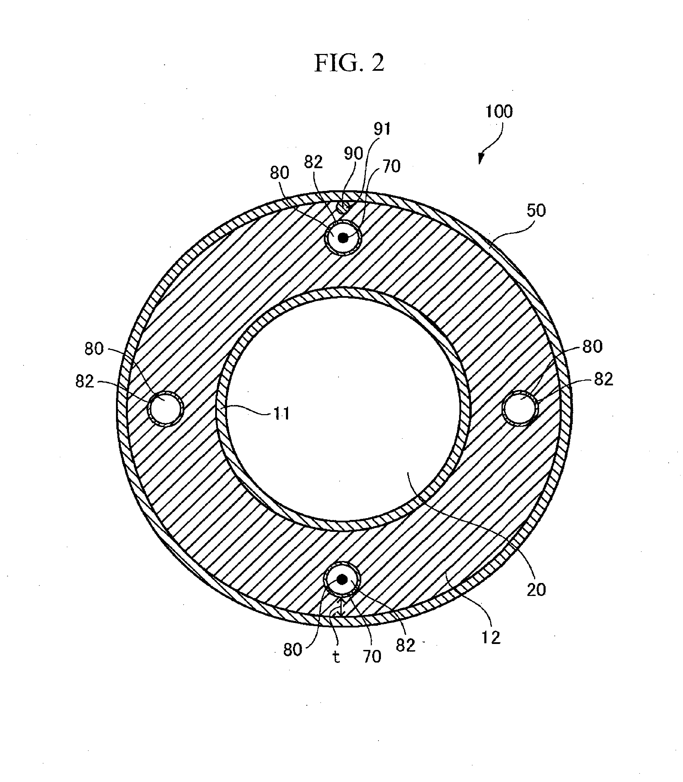

First, an outline of a catheter 100 which is a medical instrument of the present embodiment will be described with reference to Figs. 1 and 2.

Fig. 1 is a cross-sectional view of the catheter 100 along a longitudinal direction thereof. Fig. 2 is a cross-sectional view in a II-II direction of Fig. 1. In Fig. 2, a reinforcement layer 30 is not shown. In addition, in Fig. 1, in regards to a coil (first coil) 90, a wire (first wire) 91 that appears at a front side and an inner side in the drawing is shown by a dotted line, but in regards to a coil (second coil) 31, only a wire (second wire) 32 that appears at an inner side in the drawing is shown by a dotted line for clarity.

The catheter 100 of the present embodiment includes a resin tubular main body (sheath) 10, in which a main lumen 20 and a sub-lumen 80 which is disposed on an outer peripheral side of the main lumen 20 and extends along a longitudinal direction of the main lumen 20, are formed; a reinforcement layer 30 which is disposed between the main lumen 20 and the sub-lumen 80 and surrounds the main lumen 20; and a wire 91 which is disposed further on an outer peripheral surface side of the tubular main body 10 than the sub-lumen 80 and is disposed so as to surround the outer periphery of the sub-lumen 80 and the main lumen 20. The wire 91 is embedded in the tubular main body 10 and supports the outer peripheral surface of the tubular main body 10.

Next, the structure of the catheter 100 will be described in detail.

The catheter 100 includes an operation wire 70, a coating layer 50, an operation portion 60 (refer to Fig. 6), a hollow tube 82, and a marker 40 in addition to the sheath 10, the reinforcement layer 30, and the wire 91 described above.

The sheath 10 is an elongated member and includes an inner layer 11 having the main lumen 20 therein and an outer layer 12 covering the inner layer 11.

Hereinafter, the tip end of the sheath 10 and the catheter 100 will be called distal end DE, the rear end of the sheath 10 is called proximal end PE, and the rear end of the catheter 100 is called proximal end CE.

The inner layer 11 is a hollow tubular layer and is formed with the main lumen 20 extending along the longitudinal direction of the catheter 100 therein. It is possible to use a fluorine-based thermoplastic polymer material for the inner layer 11, for example. More specifically, it is possible to use polytetrafluoroethylene (PTFE), polyvinylidene fluoride (PVDF), and perfluoroalkoxy fluorine resin (PFA). With the use of the fluorine-based resin for the inner layer 11, favorable delivery performance is expected when supplying a contrast agent, a liquid medicine, or the like to a lesion portion through the main lumen 20 of the catheter 100.

The cross-sectional shape, which is orthogonal to the longitudinal direction of the catheter 100, of the main lumen 20 is a circular shape.

The outer layer 12 is a resin tubular body that covers the inner layer 11. The outer layer 12 has a thickness thicker than that of the inner layer 11 and constitutes the main wall thickness of the sheath 10.

A wide range of the thermoplastic polymer is used for the outer layer 12. For example, it is possible to use polyethylene (PE), polyamide (PA), nylon elastomers, polyurethane (PU), ethylene-vinyl acetate resin (EVA), polyvinyl chloride (PVC), polypropylene (PP), or the like in addition to polyimide (PI), polyamide-imide (PAI), and polyethylene terephthalate (PET).

The reinforcement layer 30 surrounds the inner layer 11 and is embedded in the sheath 10 in a manner of being included in the outer layer 12. The reinforcement layer 30 is used to reinforce the main lumen 20. In the present embodiment, the reinforcement layer 30 is the coil 31. It is possible to use a fine wire of a polymer fiber such as PI, PAI, or PET in addition to a metal fine wire such as stainless steel (SUS) or nickel titanium alloy, for a wire material (wire 32) that constitutes the reinforcement layer 30. In addition, the cross-sectional shape of the wire material is not particularly limited, and may be a round wire or a flat wire. However, in the present embodiment, the wire material is the round wire. In addition, the coil 31 may be configured such that the wire 32 is pitch-wound. Furthermore, the coil 31 may be tightly wound.

In the catheter 100 of the present embodiment, the sub-lumen 80 is formed inside the outer layer 12 and outside the reinforcement layer 30.

The operation wire 70 is loosely inserted into the sub-lumen 80 and extends along a longitudinal direction of the sub-lumen 80.

The operation wire 70 may be configured to have a wire. However, it is preferable that the operation wire be a stranded wire configured to have a plurality of fine wires which are stranded.

As shown in Fig. 1, the hollow tube 82 (82a, 82b) is embedded in the outer layer 12 and is disposed in the periphery (at an outer periphery side) of the main lumen 20 such that the longitudinal direction of the hollow tubes runs along the longitudinal direction of the main lumen 20.

The hollow tube 82 divides the sub-lumen 80. The hollow tube 82 dividing the sub-lumen 80 is provided along the longitudinal direction of the catheter 100. Although not shown in the drawing, the proximal end PE side of the sheath 10 is opened. In addition, the distal end of the sheath 10 of the hollow tube 82 is closed by the marker 40.

The hollow tube 82 is disposed outside the reinforcement layer 30 and protects the inside of the reinforcement layer 30, that is, the main lumen 20 from the operation wire 70 (70a, 70b) which is disposed inside the hollow tube 82.

In the present embodiment, as shown in Fig. 2, a plurality of hollow tubes 82 are provided. Specifically, the plurality of hollow tubes 82 are disposed on the same circumference so as to surround the main lumen 20. In the present embodiment, four hollow tubes 82 are disposed at regular intervals. The operation wire 70 is disposed inside a pair of hollow tubes 82 facing each other across the center of the main lumen 20. In addition, the operation wire 70 is not disposed inside the other pair of hollow tubes 82 facing each other across the center of the main lumen 20.

The number of the hollow tubes 82 and the sub-lumens 80 is not limited to four, and can be appropriately selected as necessary.

The hollow tube 82 is formed by a material which is different from the outer layer 12. With such a configuration, it is possible to set the hollow tube 82 to be formed of a material having a higher bending rigidity or tensile elastic modulus than the outer layer 12. Examples of the materials constituting the hollow tube 82 include materials such as polytetrafluoroethylene (PTFE), perfluoroalkoxy fluorocarbon resin (PFA), and tetrafluoroethylene-hexafluoropropylene copolymer (FEP). Any one or more of the materials are preferably set as main components. The sliding property of the operation wire and heat resistance can be improved using the materials.

With the use of such a hollow tube 82, the torsional rigidity of the catheter 100 is enhanced, and therefore, it is possible to prevent the sheath 10 from being topically twisted when the sheath 10 is rotated in the longitudinal direction thereof as a rotating shaft.

In addition, as shown in Fig. 1, in the distal end DE of the sheath 10, a tip end portion71 (71a, 71b) of the operation wire 70 (70a, 70b) is fixed to the distal end DE by fixing the tip end portion71 (71a, 71b) of the operation wire 70 (70a, 70b) to the marker 40. The respective operation wires 70 are slidably inserted into the sub-lumens 80 (80a, 80b). Moreover, a distal end portion 15 of the catheter 100 is bent (refer to Fig. 6) by pulling the proximal ends of the operation wires 70 (70a, 70b). In addition, in the catheter 100 of the present embodiment, the curvature and the direction of the bending distal end portion 15 are changed in various ways by selecting the operation wires 70 (70a, 70b) to be pulled.

Here, as specific materials of the operation wire 70, for example, polymer fibers such as polyether ether ketone (PEEK), polyphenylene sulfide (PPS), polybutylene terephthalate (PBT), PI, or PTFE, or metal wires such as SUS, coated steel wires having corrosion resistance, titanium, or titanium alloy can be used. It is possible to use PVDF, high-density polyethylene (HDPE), polyester, or the like in addition to the above-described materials.

Next, the wire 91 will be described.

As shown in Figs. 1 and 2, the wire 91 is embedded in the outer layer 12 and is disposed so as to surround the outer periphery of the main lumen 20.

In the present embodiment, the wire 91 is spirally wound to form the coil 90. The coil 90 extends along the longitudinal direction of the main lumen 20. In the present embodiment, the shaft of the coil 90 is coincident with the central shaft of the main lumen 20.

The cross-sectional shape of the wire 91 orthogonal to the longitudinal direction thereof is not particularly limited, and the wire 91 may be a round wire and a flat wire. However, in the present embodiment, the cross-sectional shape is the round wire and the diameter of the wire 91 is smaller than the diameter of the wire 32 constituting the reinforcement layer 30. For example, the diameter of the wire 91 is 10 µm to 30 µm.

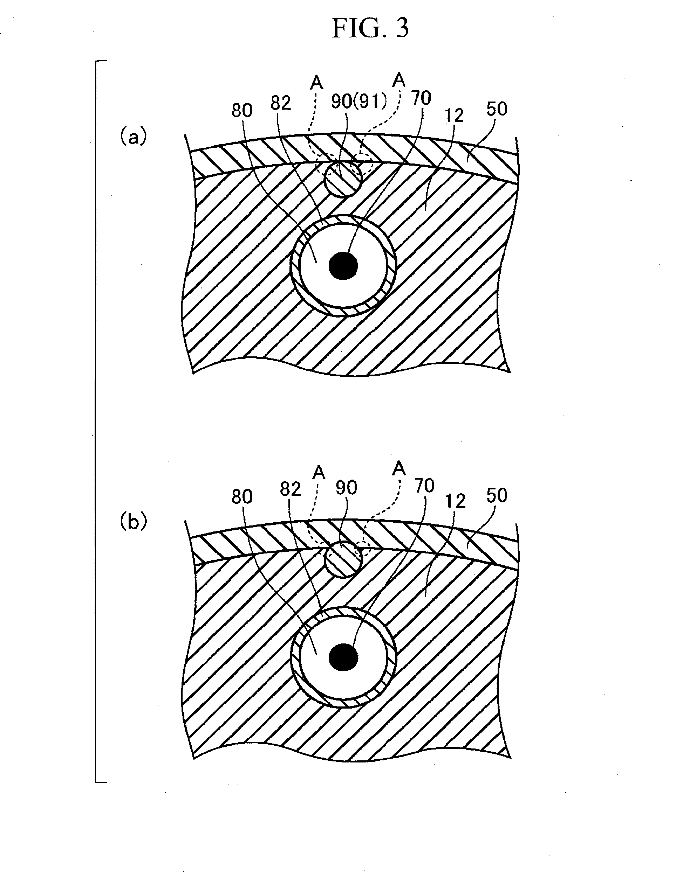

The wire 91 supports the outer peripheral surface of the sheath 10, that is, the outer peripheral surface of the outer layer 12 as shown in Figs. 3(a) and 3(b). Here, the supporting of the outer peripheral surface of the sheath 10 by the wire 91 indicates that at least a portion of the wire 91 exists immediately below the outer peripheral surface of the sheath 10 when the wire 91 is set to be on an upper side in the cross section in a direction orthogonal to the longitudinal direction of the catheter 100.

For example, in Figs. 3(a) and 3(b), in the portion A surrounded by a dotted line, a portion of the wire 91 exists immediately below the outer peripheral surface of the sheath 10 and the wire 91 supports the outer peripheral surface of the sheath 10. That is, a portion of the outer layer 12 enters a state of being upheld on a portion of the outer peripheral portion of the wire 91.

In addition, in a case where the wire 91 is the round wire, it is necessary that the wire 91 be embedded in the sheath 10 up to a position exceeding one-half of the diameter of the wire 91 in the cross section in the direction orthogonal to the longitudinal direction of the catheter 100. Among the range of the positions, it is preferable that the wire 91 be embedded in the sheath 10 up to a position with a diameter larger than or equal to two-thirds of the wire 91 in the cross section in the direction orthogonal to the longitudinal direction of the catheter 100.

In contrast, in a case where the wire 91 is a flat wire, it is necessary that at least a portion of the upper surface of the wire 91 is embedded in the sheath 10 in the cross section in the direction orthogonal to the longitudinal direction of the catheter 100.

In the present embodiment, as shown in Fig. 3(a), in the cross section in the direction orthogonal to the longitudinal direction of the catheter 100, a portion of the outer peripheral surface of the wire 91 is exposed from the sheath 10 and the outer peripheral surface of the wire 91 is flush with the outer peripheral surface of the sheath 10. However, as shown in Fig. 3(b), a portion of the outer peripheral surface of the wire 91 may be exposed from the sheath 10 and the outer peripheral surface of the wire 91 may not be flush with the outer peripheral surface of the sheath 10.

The outer peripheral surface of the wire 91 may be flush with the outer peripheral surface of the sheath 10 over the entire wire 91 in the longitudinal direction, and there may be a region in which the outer peripheral surface of the wire 91 is covered with the sheath 10 in a portion of the wire 91 in the longitudinal direction. That is, in a cross section in the direction orthogonal to the longitudinal direction of the catheter 100, the wire 91 may be completely covered with the sheath 10.

In addition, a portion in which the outer peripheral surface of the wire 91 is flush with the outer peripheral surface of the sheath 10 as shown in Fig. 3(a) and a portion in which the outer peripheral surface of the wire 91 is not flush with the outer peripheral surface of the sheath 10 as shown in Fig. 3(b) may be mixed.

Furthermore, the outer peripheral surface of the wire 91 may be covered with the outer layer 12 over the entire wire 91 in the longitudinal direction.

In the cross section in the direction orthogonal to the longitudinal direction of the catheter 100, the thickness of the sheath 10 on the wire 91 is not particularly limited in a case where the wire 91 is completely covered with the sheath 10. However, from the viewpoint of making the wall thickness of the sheath 10 thin, the thickness of the sheath 10 on the wire 91 is preferably 1 times or less than the diameter of the wire 91. Furthermore, the thickness of the sheath 10 on the wire 91 is more preferably smaller than or equal to one-half of the diameter of the wire 91, and is particularly preferably smaller than or equal to one-fifth thereof.

In addition, in the present embodiment, as shown in Figs. 3(a) and 3(b), a portion of the outer layer 12 exists between the wire 91 and the hollow tube 82 and the wire 91 does not come into contact with the hollow tube 82.

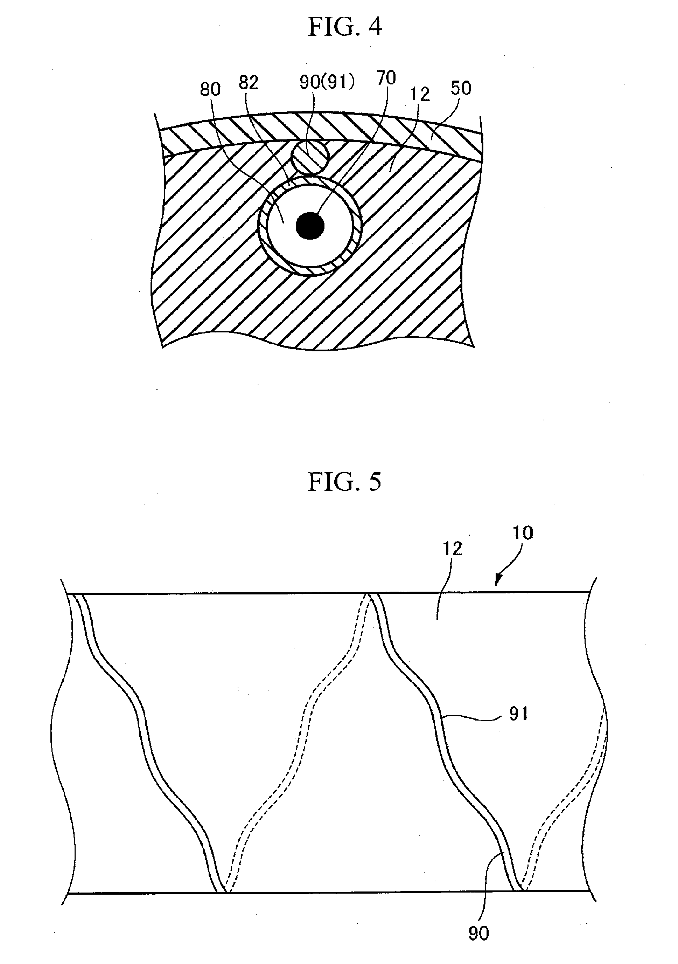

However, as shown in Fig. 4, the wire 91 may come into contact with the hollow tube 82 and may be disposed between the hollow tube 82 and the outer peripheral surface of the outer layer 12.

In addition, the elastic modulus of the wire 91 is smaller than that of the wire 32 of the reinforcement layer 30. Examples of the material constituting the wire 91 include metal having a small elastic modulus, and it is possible to use annealed material of stainless steel (SUS) or the like.

The above-described wire 91 is spirally wound to form the coil 90 surrounding the main lumen 20.

The main lumen 20 is surrounded by the coil 31 and a plurality of sub-lumens 80 are formed on an outer peripheral side of the coil 31. The coil 90 is disposed so as to surround the plurality of sub-lumens 80, the coil 31, and the main lumen 20. In other words, the sub-lumens 80 are disposed between the coil 31 and the coil 90.

Accordingly, the diameter of the coil 90 is made to be greater than the diameter of the coil 31.

The coil 90 is configured such that the wire 91 is pitch-wound. As shown in Fig. 1, the gap (pitch) P2 between the wires 91 of the coil 90 is larger than the gap (pitch) P1 between the wires 32 of the reinforcement layer 30. Here, the pitch is the distance between the centers of the width of adjacent wires in a cross section along the longitudinal direction of the catheter.

It is preferable that P2 is 2 times to 50 times P1. Among the ranges, it is preferable that P2 be 5 times or more than P1 from the viewpoint of not excessively increasing the rigidity of the catheter 100.

As shown in Fig. 1, a resin material constituting the sheath 10 is impregnated between the wires 91 (loops) of the coil 90. The outer peripheral surface of the sheath 10 is disposed between the wires 91. Accordingly, it is possible to support the outer peripheral surface of the sheath 10 between the wires 91.

In addition, as shown in Fig. 5, the wire 91 of the coil 90 is topically bent in a direction different from the winding direction of the wire 91 when the sheath 10 is viewed from a direction orthogonal to the longitudinal direction thereof. Fig. 5 is a side surface view of the sheath 10 when viewed from a direction orthogonal to the longitudinal direction. The wire 91 is generally covered with a coating layer 50, but here, the wire is represented by a solid line.

As shown in Fig. 5, the wire 91 meanders and is topically bent in the longitudinal direction of the sheath 10.

Although detailed description will be made hereinafter, when the coil 90 is disposed in a heat shrinkable tube which is heat-shrunk, the coil 90 is compressed in a radial direction and the wire 91 is topically bent as described above.

Here, in the present embodiment, the coil 90 is provided only at a distal end portion of the sheath 10, and is not provided at an intermediate portion and a proximal end portion. Only the distal end portion of the main lumen 20 is surrounded by the wire 91.

Furthermore, the catheter 100 has a marker 40. In a plan view from a direction along the longitudinal direction of the sheath 10, the coil 90 is disposed such that the marker 40 and the wire 91 of the coil 90 overlap each other.

Next, the operation portion 60 will be described.

As shown in Fig. 6, the catheter 100 includes the operation portion 60. The operation portion 60 is provided at a proximal end portion 17 of the catheter 100. In addition, the space between the distal end portion 15 and the proximal end portion 17 is called an intermediate portion 16.

The operation portion 60 includes an shaft portion 61 extending in the longitudinal direction of the catheter 100; sliders 64 (64a, 64b) that respectively advance and retreat in the longitudinal direction of the catheter 100 with respect to the shaft portion 61; a handle portion 62 axially rotating the shaft portion 61; and a holding portion 63 through which the sheath 10 is rotatably inserted. In addition, the proximal end portion 17 of the sheath 10 is fixed to the shaft portion 61. In addition, a handle portion 62 and the shaft portion 61 are integrally configured. The entire sheath 10 including the operation wire 70 is torque-driven together with the shaft portion 61 by relatively rotating the holding portion 63 and the handle portion 62.

Accordingly, the operation portion 60 of the present embodiment rotatably operates the distal end portion 15 of the sheath 10. In the present embodiment, the handle portion 62 as a rotating operation portion that torque-rotates the sheath 10 and the sliders 64 as a bending operation portion that bends the sheath 10 are integrally provided. However, the present invention is not limited thereto and the handle portion 62 and the sliders 64 may be separately provided.

The proximal end of a first operation wire 70a protrudes from the proximal end portion 17 of the sheath 10 to the base end side and is connected to a slider 64a of the operation portion 60. Similarly, the proximal end of a second operation wire 70b is also connected to a slider 64b of the operation portion 60. By separately sliding the slider 64a and the slider 64b to the base end side with respect to the shaft portion 61, the first operation wire 70a or the second operation wire 70b connected thereto is pulled, and tensile force is applied to the distal end portion 15 of the sheath 10. Accordingly, the distal end portion 15 is bent to the pulled side of the operation wire 70.

Next, the marker 40 will be described.

As shown in Fig. 1, the marker 40 is provided at the distal end DE of the sheath 10. The marker 40 is a ring-like member formed of a material having opaque radioactive rays such as X-rays. Specifically, it is possible to use metallic materials such as platinum for the marker 40. The marker 40 of the present embodiment is provided in the periphery of the main lumen 20 and inside of the outer layer 12.

The coating layer 50 is a hydrophilic layer that constitutes an outermost layer of the catheter 100. It is possible to use hydrophilic materials such as polyvinyl alcohol (PVA) and polyvinylpyrrolidone for the coating layer 50. The coating layer 50 is provided on the outer layer 12. In the present embodiment, the surface of the wire 91 exposed from the outer layer 12 is also coated.

Here, the representative size of the catheter 100 of the present embodiment will be described. It is possible to set the radius of the main lumen 20 to about 200 µm to 300 µm, the thickness of the inner layer 11 to about 10 µm to 30 µm, the thickness of the outer layer 12 to about 50 µm to 150 µm, the outer diameter (diameter) of the reinforcement layer 30 to about 500 µm to 860 µm, and the inner diameter (diameter) of the reinforcement layer 30 to about 420 µm to 660 µm. Moreover, it is possible to set the radius from the shaft center of the catheter 100 to the center of the sub-lumen 80 to about 300 µm to 350 µm, the inner diameter of the sub-lumen 80 to about 40 µm to 100 µm, and the thickness of the operation wire 70 to about 30 µm to 60 µm. In addition, the wall thickness of the hollow tube 82 can be set to about 3 µm to 15 µm. Moreover, the maximum outer diameter (radius) of the catheter 100 can be set to about 350 µm to 490 µm. In addition, the length (wall thickness) t (refer to Fig. 2) which is a length along the radial direction of the main lumen and is from the outer periphery of the sub-lumen 80 to the outer periphery of the outer layer 12 can be set to 10 µm to 100 µm.

That is, the outer diameter of the catheter 100 of the present embodiment is less than 1 mm in diameter, and it is possible to insert the catheter into the blood vessel such as the celiac artery. In addition, in relation to the catheter 100 of the present embodiment, the travelling direction is freely operated by pulling the operation wires 70 (70a, 70b). Therefore, for example, it is possible to advance the catheter 100 in a desired direction even within a branching blood vessel.

### [Operation Example]

Next, an operation example of the catheter 100 of the present embodiment will be described with reference to Fig. 6. First, in the catheter 100 of the present embodiment, when the proximal end of the operation wire 70 (the first operation wire 70a or the second operation wire 70b) is pulled, tensile force is applied to the distal end portion 15 of the catheter 100, and a portion of or the entirety of the distal end portion 15 is bent toward the side of the sub-lumen 80 (a sub-lumen 80a or a sub-lumen 80b) through which the operation wire 70 (the first operation wire 70a or the second operation wire 70b) is inserted. Meanwhile, in a case where the proximal end of the operation wire 70 is pushed to the catheter 100, a pushing force is not substantially applied to the distal end portion 15 of the catheter 100 from the operation wire 70.

The distal end portion 15 of the catheter 100 is referred to as a region which has a predetermined length and includes the distal end DE of the catheter 100. Similarly, the proximal end portion 17 of the catheter 100 is referred to as a region which has a predetermined length and includes the proximal end CE of the catheter 100. The intermediate portion 16 is referred to as a region which has a predetermined length between the distal end portion 15 and the proximal end portion 17. In addition, the bending of the catheter 100 is referred to as a portion of or the entirety of the catheter 100 being curved or bent in a zigzag manner.

In the catheter 100 of the present embodiment, the curvature of the bending distal end portion 15 is changed in various ways by selecting only the first operation wire 70a, only the second operation wire 70b, or the two operation wires 70a and 70b at the same time as (an) operation wire(s) 70 to be pulled. Accordingly, it is possible to freely advance the catheter 100 to the lumens of a living body which are branched at various angles.

The catheter 100 of the present embodiment can separately pull a plurality of proximal ends of the operation wires 70 (the first operation wire 70a or the second operation wire 70b). Moreover, it is possible to change the bending direction using the operation wires 70 to be pulled. Specifically, when the first operation wire 70a is pulled as shown in Figs. 6(b) and 6(c), the distal end portion is bent to the side provided with the first operation wire 70a. When the second operation wire 70b is pulled as shown in Figs. 6(d) and 6(e), the distal end portion is bent to the side provided with the second operation wire 70b. In addition, it is possible to change the curvature (curvature radius) of the bending by adjusting the pulling capacity of the operation wires 70 (70a, 70b). Specifically, when the first or second operation wire 70a, 70b is pulled slightly as shown in Figs. 6(b) and 6(d), the distal end portion 15 is bent with a small curvature (the curvature radius is large). In contrast, when the first or second operation wire 70a, 70b is pulled longer as shown in Figs. 6(c) and 6(e), the distal end portion 15 is bent with a large curvature (the curvature radius is small).

### [Manufacturing Example]

Next, a method of manufacturing the catheter 100 of the present embodiment will be described.

First, an outline of the method of manufacturing the catheter 100 will be described.

The method of manufacturing the catheter 100 of the present embodiment includes a step of preparing a molded body having a resin sheath 10 in which a main lumen formation region and a sub-lumen formation region which is disposed on an outer peripheral side of the main lumen formation region and extends along the longitudinal direction of the main lumen formation region, are formed, and a reinforcement layer 30 which is disposed between the main lumen formation region and the sub-lumen formation region and surrounds the main lumen formation region; a step of disposing a wire 91 on an outer peripheral side of the sheath 10 of the molded body so as to surround the tubular main body 10; and a step of embedding the wire 91 in the sheath 10 such that the wire 91 supports the outer peripheral surface of the sheath 10 by pressurizing the molded body and the wire 91 from a radial direction of the main lumen formation region of the sheath 10 and by heating the molded body and the wire 91.

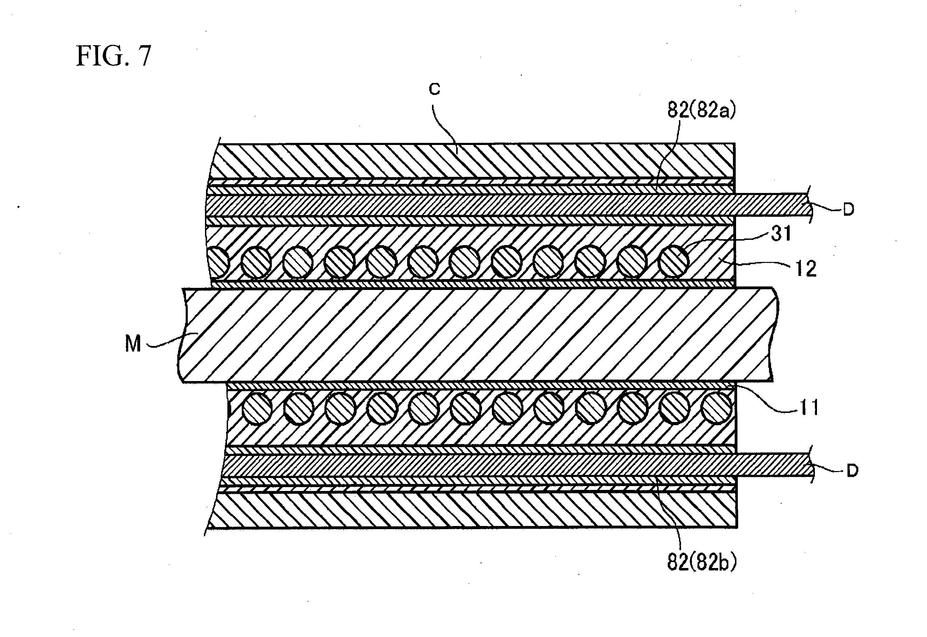

Next, the method of manufacturing the catheter 100 will be described in detail with reference to Fig. 7.

First, the inner layer 11 is extruded to the periphery of a mandrel M. Then, the coil 31 is provided in the periphery of the inner layer 11. On the other hand, the outer layer 12 is extruded and molded in advance. At this time, the outer layer 12 is extruded and molded while discharging fluid such as gas so as to form a hole through which the hollow tube 82 is inserted.

The hollow tube 82 is also made by extruding and molding a material containing resin that constitutes the hollow tube. The hollow tube is extruded and molded while discharging fluid such as gas with respect to the material of the hollow tube 82 so as to form an elongated hollow portion along the longitudinal direction.

Thereafter, the outer layer 12 is covered on the periphery of the coil 31 in a state where the coil 31 is covered on the periphery of the inner layer 11. Furthermore, the hollow tube 82 is inserted into the outer layer 12. At this time, a core wire D is inserted into the hollow tube 82 in advance in order to suppress deformation of the hollow tube 82. The core wire D has a diameter larger than that of the operation wire.

Next, a heat shrinkable tube C is covered on the periphery of the outer layer 12. The outer layer 12, the coil 31, the inner layer 11, and the hollow tube 82 are pressurized from the outside toward the radial direction of the inner layer 11 by shrinking the heat shrinkable tube by heating. In addition, the outer layer 12 is melted by the heating. The heating temperature is higher than the melting temperature of the outer layer 12 and is lower than the melting temperature of the inner layer 11 and the hollow tube 82. The outer layer 12 and the inner layer 11 are joined to each other by welding through the heating. At this time, the material constituting the outer layer 12 includes the coil 31 and the coil 31 is impregnated in the outer layer 12.

Next, the heat shrinkable tube is removed from the outer layer 12 by making an incision on the heat shrinkable tube C and by tearing the heat shrinkable tube C.

The coil 90 is covered on the periphery of the outer layer 12 of the molded body which is obtained as described above. Specifically, the above-described molded body is inserted into the coil 90.

Then, the heat shrinkable tube is covered on the periphery of the coil 90 again. The coil 90, the outer layer 12, the coil 31, the inner layer 11, and the hollow tube 82 are pressurized from the outside toward the radial direction of the inner layer 11 by shrinking the heat shrinkable tube. In addition, the outer layer 12 is melted by the heating. Accordingly, the coil 90 is embedded in the outer layer 12, and the outer layer 12 is impregnated between the wires 91 (between loops) of the coil 90. The heating temperature is higher than the melting temperature of the outer layer 12 and is lower than the melting temperature of the inner layer 11 and the hollow tube 82.

Here, the inner diameter of the coil 90 becomes small since the coil 90 is pressurized in the radial (winding diameter) direction thereof by the heat shrinkable tube. For this reason, the wire 91 is topically bent as described above.

Then, the heat shrinkable tube is torn to be removed from the outer layer 12.

Next, the core wire D which is inserted into the hollow tube 82 is pulled out and the operation wire 70 is inserted into the hollow tube 82.

Then, fixing of the tip end portion of the operation wire 70 with respect to the marker 40 and caulking and fixing of the marker 40 with respect to the periphery of the tip end portion of the outer layer 12 are performed.

Next, the mandrel M in the inner layer 11 is pulled out. The pulling out of the mandrel is performed in a state in which the diameter of the mandrel M is made fine by pulling both the ends of the mandrel in the longitudinal direction. Accordingly, a hollow which becomes the main lumen 20 is formed in the center of the inner layer 11.

Next, the base end portion of the operation wire 70 is connected to the operation portion 60 which is separately made.

Next, the coating layer 50 is formed.

As described above, it is possible to obtain the catheter 100.

Next, the effect of the present embodiment will be described.

In the present embodiment, the wire 91 is disposed further on the outer peripheral surface side of the sheath 10 (on a side opposite to the main lumen 20 side from the sub-lumen 80) than the sub-lumen 80. The wire 91 is embedded in the sheath 10 and supports the outer peripheral surface of the sheath 10. The outer peripheral surface is elevated by the wire 91 supporting the outer peripheral surface of the sheath 10, and therefore, it is possible to secure the wall thickness of the partial sheath 10 positioned further on the outer peripheral surface side of the sheath 10 than the sub-lumen 80. Accordingly, it is possible to protect the sub-lumen 80.

It is possible to prevent the intensity of the peripheral wall of the sub-lumen 80 from being insufficient, for example, when pulling the operation wire 70 by protecting the sub-lumen 80 in this manner.

In addition, it is possible to reliably dispose the sub-lumen 80 in the sheath 10 by securing the thickness of the sheath 10 of the portion disposed further on the outer peripheral surface side of the sheath 10 than the sub-lumen 80. Therefore, it is possible to obtain the catheter 100 which is excellent in manufacturing stability.

Furthermore, in the present embodiment, the wire 91 is spirally wound to form a coil shape. Accordingly, it is possible to suppress variations in bending rigidity depending on the rotational angle in the circumferential direction of the catheter 100.

In addition, in the present embodiment, the coil 90 is provided only at the distal end portion of the sheath 10. For this reason, it is possible to prevent the rigidity of the catheter 100 from being unnecessarily increased.

In addition, the diameter of the sheath 10 is reduced from the proximal end side to the distal end side, and the wall thickness of the sheath 10 is particularly thin at the distal end portion. For this reason, it is possible to reliably protect the sub-lumen 80 by providing the coil 90 at the distal end portion of the sheath 10.

Furthermore, in the present embodiment, the diameter of the wire 91 is smaller than the diameter of the wire 32 constituting the reinforcement layer 30. It is possible to suppress the rigidity of the catheter 100 from being unnecessarily increased by reducing the diameter of the wire 91 in this manner.

In addition, the elastic modulus of the wire 91 is smaller than that of the wire 32 of the reinforcement layer 30. By reducing the elastic modulus of the wire 91, it is possible to obtain the catheter 100 which is not prevented from being bent.

Furthermore, the pitch (gap) P2 between the wires 91 of the coil 90 is larger than the pitch (gap) P1 between the wires 32 of the reinforcement layer 30. By doing this, it is possible to prevent the rigidity of the catheter 100 from being unnecessarily increased.

The resin material constituting the sheath 10 is impregnated between the wires 91 of the coil 90. The outer peripheral surface of the sheath 10 is disposed between the wires 91. Accordingly, it is possible to support the outer peripheral surface of the sheath 10 between the wires 91.

In addition, in the present embodiment, as shown in Fig. 5, the wire 91 of the coil 90 is topically bent in a direction different from the winding direction of the wire 91 when the sheath 10 is viewed from a direction orthogonal to the longitudinal direction thereof. The wire 91 is anchored on the outer layer 12 by the wire 91 being bent in this manner. For this reason, it is possible to rigidly fix the wire 91 and the outer layer 12 to each other.

In addition, in the present embodiment, in a plan view from a direction along the longitudinal direction of the sheath 10, the coil 90 is disposed such that the marker 40 and the wire 91 of the coil 90 overlap each other. Accordingly, it is possible to reliably prevent the coil 90 from being further moved to the distal end side than the marker 40.

Furthermore, in the present embodiment, the coil 90 is provided on the outer peripheral side of the sub-lumen 80 so as to surround the sub-lumen 80. It is possible to protect the lumen of a living body from the operation wire 70 disposed inside the sub-lumen 80 using the coil 90.

When the wall thickness of the outer layer 12 is thin, there is a possibility that the outer shape of the cross section in a direction orthogonal to the longitudinal direction of the sheath 10 may be a substantially square shape which is obtained by connecting the hollow tubes 82. However, by disposing the coil 90 surrounding the main lumen 20 outside the sub-lumen 80, even when the wall thickness of the outer layer 12 is thin, the outer peripheral surface of the outer layer 12 is supported by the coil 90 and it is possible to make the outer shape of the cross section in a direction orthogonal to the longitudinal direction of the sheath 10 a round shape. Accordingly, it is possible to suppress an occurrence of variations of bending rigidity depending on the rotational angle in the circumferential direction of the catheter 100.

Furthermore, in the present embodiment, the wire 91 is provided in the outer layer 12 of the sheath 10. The wire 91 is disposed on the outer peripheral side of the main lumen 20 and is disposed further on the outer peripheral surface side of the sheath 10 than the sub-lumen 80 (hollow tube 82). That is, the wire 91 is disposed so as to surround the plurality of sub-lumens 80 (hollow tubes 82) and the main lumen 20.

By providing such a wire 91, it is possible to prevent the position of the hollow tubes 82 from being deviated further to the outer peripheral side than the wire 91, and it is also possible to prevent large positional deviation with respect to the main lumen 20 of the hollow tubes 82. Accordingly, it can be said that the catheter 100 of the present embodiment has a shape which is excellent in manufacturing stability.

Furthermore, in the present embodiment, the outer layer 12, the coil 31, the inner layer 11, and the hollow tube 82 are pressurized by the heat shrinkable tube (first pressurization) and molded. Then, the coil 90 is disposed in the periphery of the obtained molded body, and the pressurization is performed again using the heat shrinkable tube (second pressurization).

Even if the position of the hollow tube 82 with respect to the main lumen 20 is deviated to the outer peripheral side of the sheath 10 than a desired position during the first pressurization, it is possible to push the hollow tube 82 to the main lumen 20 side using the coil 90 during the second pressurization.

Accordingly, it is possible to position the hollow tube 82 at a desired position with respect to the main lumen 20. From this point, it can also be said that the catheter 100 of the present embodiment has a shape which is excellent in manufacturing stability.

### (Second Embodiment)

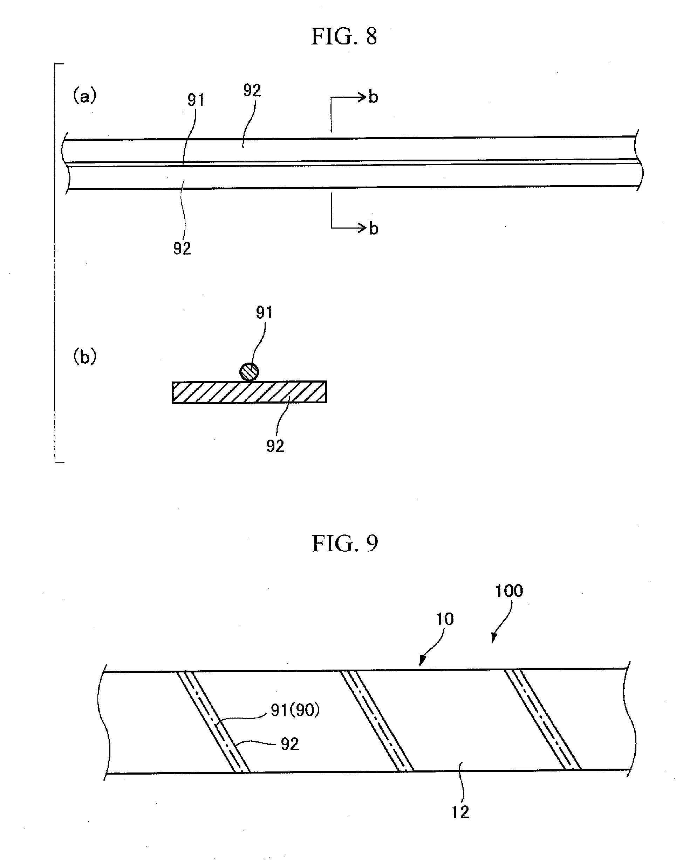

Next, a method of manufacturing a catheter 100 different from the first embodiment will be described with reference to Figs. 8 and 9.

First, in the present embodiment, similar to the first embodiment, a molded body having an outer layer 12, a coil 31, an inner layer 11, and a hollow tube 82 are prepared. Specifically, similar to the first embodiment, the outer layer 12 is covered on the periphery of the coil 31 in a state where the coil 31 is covered on the periphery of the inner layer 11. Furthermore, the hollow tube 82 is inserted into the outer layer 12.

Next, a heat shrinkable tube is covered on the periphery of the outer layer 12. The outer layer 12, the coil 31, the inner layer 11, and the hollow tube 82 are pressurized from the outside toward the radial direction of the inner layer 11 by shrinking the heat shrinkable tube by heating.

Then, the heat shrinkable tube is removed from the outer layer 12 by making an incision on the heat shrinkable tube and by tearing the heat shrinkable tube. The molded body is obtained as described above. The molded body includes a main body portion (the inner layer 11, the outer layer 12, and the hollow tube 82) which becomes a sheath 10 in which a main lumen formation region and a sub-lumen formation region which is disposed at an outer peripheral side of the main lumen formation region and extends along the longitudinal direction of the main lumen formation region, are formed; and a reinforcement layer 30.

Meanwhile, a wire 91 with resin tape 92 as shown in Figs. 8(a) and 8(b) is prepared. Fig. 8(a) is a plan view of the wire 91 with the resin tape 92. Fig. 8(b) is a cross-sectional view in the b-b direction of Fig. 8(a) and a cross-sectional view in a direction orthogonal to the longitudinal direction of the wire 91 with the resin tape 92.

The resin tape 92 has an elongated shape extending along the longitudinal direction of the wire 91. The wire 91 is fixed in the center of the tape 92 in the width direction thereof.

The wire 91 may be embedded in the resin tape 92 and may be exposed from the surface of the resin tape 92. In the present embodiment, the wire 91 is exposed from the surface of the resin tape 92.

As the resin constituting the resin tape 92, for example, it is possible to use polyethylene (PE), polyamide (PA), nylon elastomers, polyurethane (PU), ethylene-vinyl acetate resin (EVA), polyvinyl chloride (PVC), or polypropylene (PP) in addition to polyimide (PI), polyamide-imide (PAI), and polyethylene terephthalate (PET). The resin tape 92 may be formed of the same material as that of the outer layer 12, and may be formed of a different material from that of the outer layer.

Such a wire 91 with the resin tape 92 is plastically deformed and is wound in a helical shape around the outer layer 12 of the molded body. At this time, the coil 90 is formed by being wound around the molded body such that the wire 91 side of the wire 91 with the resin tape 92 becomes the outer layer 12 side.

Thereafter, the heat shrinkable tube is covered on the periphery of the wire 91 with the resin tape 92 again. The wire 91 with the resin tape 92, the outer layer 12, the coil 31, the inner layer 11, and the hollow tube 82 are pressurized from the outside toward the radial direction of the inner layer 11 by shrinking the heat shrinkable tube. In addition, the outer layer 12 and the resin tape 92 are melted by the heating. Accordingly, the outer peripheral portion of the sheath 10 is configured to have the outer layer 12 and the resin tape 92.

The coil 90 is embedded in the outer peripheral portion of the sheath 10 and a resin material (mainly, a resin material constituting the outer layer 12) is impregnated between the wires 91 (between the loops) of the coil 90. The heating temperature is higher than the melting temperature of the outer layer 12 and the resin tape 92 and is lower than the melting temperature of the inner layer 11 and the hollow tube 82.

Here, the inner diameter of the coil 90 becomes small since the coil 90 is pressurized in the radial (winding diameter) direction thereof by the heat shrinkable tube. For this reason, similar to the first embodiment, the wire 91 is topically bent in a direction different from the winding direction. However, in the present embodiment, the coil is constituted by the wire 91 and the molded body is not inserted into the coil, but the wire 91 is wound around the molded body. For this reason, there is a possibility that the shrinkage factor of the inner diameter of the coil when being pressurized by the heat shrinkable tube may become small compared to the first embodiment. For this reason, there is a possibility that the curve of the wire 91 becomes smaller than that of the first embodiment.

Then, the heat shrinkable tube is torn to be removed.

The process thereafter is the same as that of the first embodiment.

In the catheter 100 which is obtained as described above, as shown in Fig. 9, the sheath 10 has the wire 91 on the outer peripheral portion thereof and a resin layer (resin tape 92) which is wound in a coil shape.

In a case where the resin tape 92 and the outer layer 12 are formed of the same material, in some cases, the resin tape 92 and the outer layer 12 are melted and mixed, and thereby, there is no boundary between the resin tape and the outer layer.

Fig. 9 is a side surface view when seen from a direction orthogonal to the longitudinal direction of the catheter 100, and the coating layer 50 is omitted from the drawing.

According to the second embodiment, it is possible to obtain the following effect in addition to obtaining the same effect as that of the first embodiment.

In the present embodiment, the wire 91 with the resin tape 92 is wound in a helical shape around the outer layer 12 of the molded body, and its manufacturing process is easier than the case where the molded body is inserted into the coil by making the wire 91 into the coil shape in advance.

The wire 91 is provided with the resin tape 92, and therefore, it is possible to prevent the wire 91 from being excessively exposed from the sheath 10 by winding the wire 91 with the resin tape around the molded body, and it is also possible to reliably support the outer peripheral surface of the sheath 10 using the wire 91.

The present invention is not limited to the above-described embodiments and modification, improvement, and the like within the scope that can achieve the purpose of the present invention is included in the present invention.

For example, in the embodiments, the operation wire 70 is inserted into the sub-lumen 80, but the operation wire may be omitted. For example, a member such as a guide wire may be inserted into the sub-lumen 80.

Furthermore, in the embodiments, the coil 90 is provided only at the distal end portion of the sheath 10 and is not provided at the intermediate portion or the proximal end portion. However, the coil 90 may be provided over the entire sheath 10.

In addition, in the embodiments, the coil 90 is constituted by spirally winding the wire 91. However, instead of the coil, a braid (mesh) formed by braiding wire 91 may be constituted by spirally winding the wire 91. Even in this case, the braid surrounds the main lumen 20 and extends along the longitudinal direction of the main lumen 20.

In addition, in the embodiments, the outer layer 12, the coil 31, the inner layer 11, and the hollow tube 82 are pressurized by the heat shrinkable tube and form a molding. Then, the wire 91 is disposed in the periphery of the molded body, and the pressurization is performed again using the heat shrinkable tube. However, it is also possible to manufacture the molded body using other manufacturing methods.

For example, similar to the embodiments, the outer layer 12 is covered on the periphery of the coil 31 in a state where the coil 31 is covered on the periphery of the inner layer 11. Furthermore, the hollow tube 82 is inserted into the outer layer 12. Furthermore, the wire 91 which is spirally wound so as to surround the periphery of the outer layer 12 is disposed, and the outer layer 12, the coil 31, the inner layer 11, the hollow tube 82, and the wire 91 are inserted into the heat shrinkable tube. Then, the outer layer 12, the coil 31, the inner layer 11, the hollow tube 82, and the wire 91 are pressurized from the outside toward the radial direction of the inner layer 11 by shrinking the heat shrinkable tube by heating. Similar to the embodiments, the coil 31 is impregnated in the outer layer 12 by melting the outer layer 12, and the outer layer 12 is further impregnated between the wires 91 of the coil 90.

### Industrial Applicability

The present invention can be applied to a medical instrument which is required to reliably protect a sub-lumen while making the wall thickness of a catheter thin, and to a method of manufacturing the medical instrument.

### Reference Signs List

- 10: Sheath (Tubular main body)
- 11: Inner layer
- 12: Outer layer
- 15: Distal end portion
- 16: Intermediate portion
- 17: Proximal end portion
- 20: Main lumen
- 30: Reinforcement layer
- 31: Coil
- 32: Wire
- 40: Marker
- 50: Coating layer
- 60: Operation portion
- 61: Shaft portion
- 62: Handle portion
- 63: Holding portion
- 64: Slider
- 64a: Slider
- 64b: Slider
- 70: Operation wire
- 70a: First operation wire
- 70b: Second operation wire
- 71: Tip end portion
- 71a: Tip end portion
- 71b: Tip end portion
- 80: Sub-lumen
- 80a: Sub-lumen
- 80b: Sub-lumen
- 82: Hollow tube
- 82a: Hollow tube
- 82b: Hollow tube
- 90: Coil
- 91: Wire
- 92: Resin tape
- 100: Catheter
- A: Portion
- C: Heat shrinkable tube
- CE: Proximal end
- D: Core wire
- DE: Distal end
- M: Mandrel
- P1: Pitch
- P2: Pitch
- PE: Proximal end

## Claims

1. A medical instrument comprising:
a resin tubular main body, in which a main lumen and a sub-lumen which is disposed on an outer peripheral side of the main lumen and extends along a longitudinal direction of the main lumen, are formed;
a reinforcement layer which is disposed between the main lumen and the sub-lumen and surrounds the main lumen; and
a first wire which is disposed further on an outer peripheral surface side of the tubular main body than the sub-lumen and is disposed so as to surround the sub-lumen and the main lumen;
wherein the first wire is embedded in the tubular main body and supports the outer peripheral surface of the tubular main body.

2. The medical instrument according to claim 1,
wherein the first wire is spirally wound and is disposed so as to surround the sub-lumen and the main lumen.

3. The medical instrument according to claim 2,
wherein the first wire constitutes a coil and a resin material constituting the tubular main body is impregnated between loops of the coil.

4. The medical instrument according to any one of claims 1 to 3,
wherein a distal end portion of the main lumen is surrounded by the first wire; and
wherein a proximal end portion of the main lumen is not surrounded by the first wire.

5. In the medical instrument according to any one of claims 1 to 3,
wherein the first wire is wound in a coil shape to form a first coil, the reinforcement layer is formed of a second coil in which a second wire is wound in the coil shape, and the diameter of the first wire of the first coil is smaller than that of the second wire of the second coil.

6. The medical instrument according to any one of claims 1 to 3,
wherein the first wire is wound in a coil shape to form a first coil;
wherein the reinforcement layer is formed of a second coil in which a second wire is wound in the coil shape; and
wherein an elastic modulus of the first wire of the first coil is smaller than that of the second wire of the second coil.

7. The medical instrument according to any one of claims 1 to 3,
wherein the first wire is wound in a coil shape to form a first coil;
wherein the reinforcement layer is formed of a second coil in which a second wire is wound in the coil shape; and
wherein the pitch of the first wire of the first coil is larger than that of the second wire of the second coil.

8. The medical instrument according to any one of claims 1 to 3,
wherein a surface of the first wire is exposed from the outer peripheral surface of the tubular main body.

9. The medical instrument according to claim 8,
wherein the surface of the first wire is flush with the outer peripheral surface of the tubular main body.

10. The medical instrument according to any one of claims 1 to 3,
wherein the first wire is wound to form a coil and is disposed so as to surround the sub-lumen and the main lumen, and the first wire is topically bent in a direction different from the winding direction of the first wire when the tubular main body is viewed from a direction orthogonal to a longitudinal direction thereof.

11. The medical instrument according to any one of claims 1 to 3,
wherein a marker is embedded in a region which is at a distal end portion of the tubular main body and is further on the distal end side than the first wire; and
wherein the marker and the wire overlap each other in a plan view from a direction along the longitudinal direction of the tubular main body.

12. The medical instrument according to any one of claims 1 to 3,
wherein a hollow tube that divides the sub-lumen is disposed inside the tubular main body; and
wherein the first wire is disposed so as to surround the hollow tube and the main lumen.

13. The medical instrument according to any one of claims 1 to 3, further comprising:
an operation wire inserted into the sub-lumen;
wherein a distal end of the tubular main body is bent by pulling the proximal end of the operation wire.

14. The medical instrument according to any one of claims 1 to 3,
wherein the first wire is wound in a coil shape to form a first coil; and
wherein the tubular main body has the first wire on the outer peripheral portion thereof and a resin layer which is wound in the coil shape.

15. The medical instrument according to any one of claims 1 to 3,
wherein the medical instrument is a catheter.

16. A method of manufacturing a medical instrument comprising:
a step of preparing a molded body having a main body portion which is a tubular resin main body and in which a main lumen formation region and a sub-lumen formation region which is disposed on an outer peripheral side of the main lumen formation region and extends along a longitudinal direction of the main lumen formation region, are formed, and a reinforcement layer which is disposed between the main lumen formation region and the sub-lumen formation region and surrounds the main lumen formation region;
a step of disposing a first wire on an outer peripheral side of the main body portion of the molded body so as to surround the molded body; and
a step of embedding the first wire in the tubular main body such that the wire supports the outer peripheral surface of the tubular main body by pressurizing the molded body and the first wire from a radial direction of the main lumen formation region of the main body portion and by heating the molded body and the first wire.

17. The method of manufacturing the medical instrument according to claim 16,
wherein, in the step of preparing the molded body, a hollow tube in which the sub-lumen formation region is formed therein is disposed in the main body portion.

18. The method of manufacturing the medical instrument according to claim 16 or 17, further comprising:
a step of preparing a wire with a tape including an elongated resin tape and a first wire which is provided on the tape and extends along with the tape;
wherein, in the step of disposing the first wire, the wire with the tape is spirally wound around the outer peripheral surface of the main body portion;
wherein, in the step of embedding the first wire in the tubular main body, the first wire is embedded in the outer peripheral surface of the tubular main body configured to have the tape and the main body portion by pressurizing the molded body and the wire with the tape from a radial direction of the main lumen formation region and by heating the molded body and the first wire.
